Europäisches Patentamt

European Patent Office

Office européen des brevets

(19)

(11) Veröffentlichungsnummer: **0 084 874**
**B1**

# EUROPÄISCHE PATENTSCHRIFT

(12)

(45) Veröffentlichungstag der Patentschrift:
**19.03.86**

(21) Anmeldenummer: **83100542.6**

(22) Anmeldetag: **21.01.83**

(51) Int. Cl.⁴: **G 01 N 33/49, G 01 N 27/06**

(54) Vorrichtung zur Bestimmung des Hämatokritwertes.

(30) Priorität: **23.01.82 DE 3202067**

(43) Veröffentlichungstag der Anmeldung:
**03.08.83 Patentblatt 83/31**

(45) Bekanntmachung des Hinweises auf die Patenterteilung:
**19.03.86 Patentblatt 86/12**

(84) Benannte Vertragsstaaten:
**AT BE CH DE FR GB IT LI LU NL SE**

(56) Entgegenhaltungen:
**DE - B - 2 729 131**

(73) Patentinhaber: **Kiesewetter, Holger Dr.,
Schneebergweg 211, D-5100 Aachen (DE)**

(72) Erfinder: **Myrenne, Heinz, Steffengasse 9,
D-5106 Roetgen (DE)**
Erfinder: **Lazar, Hartmut, Wiesental 29, D-5100 Aachen
(DE)**
Erfinder: **Mussler, Klaus, Im Grüntal 66, D-5100 Aachen
(DE)**
Erfinder: **Kiesewetter, Holger, Dr., Schneebergweg 211,
D-5100 Aachen (DE)**

(74) Vertreter: **KUHNEN & WACKER Patentanwaltsbüro,
Schneggstrasse 3-5 Postfach 1729, D-8050 Freising (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Vorrichtung zur Bestimmung des Hämatokritwertes gemäß dem Oberbegriff des Anspruchs 1.

Eine Vorrichtung der eingangs erwähnten Art ist beispielsweise aus der DE-B2-2729131 bekannt. In dieser Meßanordnung und der der DD-A-78307 sind die Elektroden so angeordnet, daß die Feldlinien senkrecht zur Sedimentationsrichtung des Bluts verlaufen. Dies ist grundsätzlich nachteilig, da der Impedanzwert vom Sedimentationsgrad abhängt und der Meßfehler um so größer wird, je größer die Sedimentationsrate ist. Vor allem bei pathologischem Blut bei dem die Aggregationsrate erhöht ist, ist dies nachteilig.

Eine einfache Korrektur der Meßgröße, die durch den Sedimentationseinfluß verfälscht wird, kann an einer derartigen Vorrichtung nicht vorgenommen werden, da die Komplexität des Vorgangs dies nicht zuläßt.

Weiterhin können die Elektroden in der Meßkammer nicht vollständig an ihren Oberflächen gereinigt werden, so daß sich auf den Elektrodenoberflächen Proteinschichten, insbesondere aus Fibrinogen, ausbilden, die den Impedanzwert und somit den Meßwert stark verfälschen.

Erschwerend kommt bei dieser Anordnung noch eine schlechte Handhabbarkeit hinzu. Durch das Einsetzen der Meßkapillare zwischen den Meßklemmen ergeben si verfälschungen durch partiellen Blutverlust aus der Meß-kapillare, wodurch der gemessene Impedanzwert durch Lufteinschlüsse stark ansteigt. Durch herausgeflossenes Blut wird die Meßapparatur verschmutzt und es können sich neue Fehlerquellen einstellen.

Infolge des nicht definierten Füllvolumens besteht weiterhin eine starke Abhängigkeit des Meßwertes vom jeweiligen Füllvolumen, da die Feldliniendichte im Blut in Abhängigkeit vom Füllvolumen variiert

Aus der DE-B- 1 229 760 ist ebenfalls eine Vorrichtung zur Bestimmung des Hämatokritwerts bekannt, bei der wiederum die Elektrodenanordnung Feldlinien erzeugt, die senkrecht zur Sedimentationsrichtung verlaufen, so daß sich die bereits vorstehend erwähnten Meßschwierigkeiten ergeben.

Die Reinigung der Elektrodenoberflächen ist ähnlich schwierig, so daß sich ähnliche Verschmutzungsprobleme bilden.

Desweiteren beruht die Meßwertanzeige auf einer linearen Abhängigkeit der Impedanz vom Hämatokritwert, die tatsächlich jedoch nicht vorliegt. Insofern beruhen die Meßwertwandlungen auf einer falschen Voraussetzung.

Schließlich kann die Meßtemperatur infolge fehlender Thermostatisierung nicht konstant gehalten werden, so daß auch hier Meßprobleme auftreten können.

Eine weitere Vorrichtung zur Bestimmung des Hämatokrits ist aus der DE-A- 21 03 285 bekannt, bei der ebenfalls die Feldlinien senkrecht zur Sedimentationsrichtung verlaufen, so daß wiederum die vorstehend erwähnten Meßschwierigkeiten auftreten. Darüberhinaus sind erhebliche Mengen Blut zur Messung notwendig. Weiterhin sind die Elektroden infolge ihrer stationären Anordnung schlecht zu reinigen, so daß wiederum die vorstehend erwähnten Aussagen gelten.

Auch in der US-A-39 22598 ist ein Hämatokritmeßgerät beschrieben, bei dem entweder wiederum die Feldlinien senkrecht zur Sedimentationsrichtung verlaufen oder aber gemäß einer anderen Ausführungsform übereinander parallel angeordnete Elektroden in Blut eingetaucht werden müssen. Bei letzterer Elektrode kann jedoch die Eintauchtiefe und damit die Dichte des Bluts nicht bestimmt werden, die zum Boden hin infolge Sedimentation zunimmt.

Weiterhin ist das Meßvolumen dieser Elektroden zu groß und eine vollständige Reinigung nur schwer möglich, so daß wiederum die vorstehend erläuterten Fehlerquellen auftreten können.

Der Erfindung liegt daher die Aufgabe zugrunde, eine Vorrichtung der eingangs erwähnten Art zur Verfügung zu stellen, bei der die Sedimentation den Meßwert nahezu unbeeinflußt läßt.

Der Erfindung liegt weiterhin die Aufgabe zugrunde, eine Vorrichtung zur Verfügung zu stellen, die leicht handhabbar und insbesondere leicht zu reinigen ist.

Die Lösung der Aufgabe erfolgt durch die kennzeichnenden Merkmale des Anspruchs 1.

Durch die Anordnung der Elektroden in der erfindungsgemäßen Vorrichtung sind die Feldlinien in Richtung der Sedimentation ausgerichtet. Eine Vielzahl von Versuchsreihen sowohl an gesunden als auch pathologischen, also stark aggregierenden bzw. sedimentierenden Bluten hat ergeben, daß hei der hier gewählten Elektrodenpositionierung die Sedimentation den Meßwert nahezu unbeeinflußt läßt und es daher zu keiner verfälschung der Meßergebnisse kommt.

Infolge der Anordnung der Elektroden in den Ebenen der Unterseite des Oberteils und der Oberseite des Unterteils ist weiterhin gewährleistet daß die Meßkammer vollständig und einfach gereinigt werden kann, so daß Meßfehler durch Verunreinigungen der Elektroden minimiert werden.

Weiterhin reichen bereits sehr kleine Blutmengen aus, um den Hämatokritwert mit hoher Genauigkeit zu bestimmen. Dabei ist das Einfüllen der Blutprobe, beispielsweise in einer Menge von 70 - 200 µl, sehr einfach. Diese Mengen die etwa einem bis drei Blutstropfen entsprechen, können an beliebiger Stelle dem Körper des Patienten entnommen werden.

Durch eine sofortige optische Kontrolle ist zu erkennen, ob das Blut nach dem Benetzen der Bodenelektrode beim Füllvorgang Luftblasen enthält, was zu einem sehr hohen Hämatokritwert führt. Insofern ist bei diesen hohen Meß-wcrten eine Wiederholung der Messung angebracht.

Wegen der sehr kurzen Meßzeit ist auch keine zusätzliche Behandlung des Bluts, wie das Vermischen mit die Koagulation hemmenden Mitteln, mehr notwendig, so daß unmittelbar nach dem Auftropfen der Bluttropfen auf die Elektrode der Meßvorgang eingeleitet werden kann.

Es ist lediglich zu beachten, daß die Mindestfüllmenge eingehalten wird, wobei durch die Anordnung der Elektroden in der Klappkammer ein konstantes Volumen zwischen den Elektroden gewährleistet wird. Sind diese Bedingungen eingehalten und beide Elektrodenoberflächen benetzt, so wurde experimentell eine Unabhängigkeit des Meßwertes von der Füllmenge festgestellt.

Der Meßvorgang kann dann eingeleitet werden, da eine elektrische Verbindung zwischen den beiden Elektroden über die Blutstropfen hergestellt worden ist.

Durch die bevorzugte Frequenz von 2 - 3 kHz werden Polarisationseffekte an den Elektroden unterdruckt. weiter wird bei dieser Frequenz der dielektrischen Eigenschaft des Plasmas als grobdisperse Lösung großer Dipolmoleküle Rechnung getragen. Man befindet sich nämlich bei dieser Frequenz außerhalb des Frequenzbereiches, in dem die sogen. Alpha-Dispersion auftritt. In diesenm Bereich spielen Zellmembranen als Konduktorelemente die vorherrschende Rolle. Zum anderen liegt die Frequenz in dem Bereich, in welchem die Beta-Dispersion gerade beginnt und die dielektrische Eigenschaft des plasmas den Meßwert gerade noch beeinflußt. Durch die Wahl dieses Frequenzwertes wurde erreicht, daß sowohl der Impedanzwert des normalen als auch des pathologischen Plasmas, in dem die Eiweißkonzentration verändert ist, nahezu gleich ist. Ebenfalls wurde durch die Wahl der hier verwendeten Frewuenzen der Einfluß des kapazitiven Widerstands der Erythrozytenmembran so weit unterdrückt, daß selbst Schwankungen im mittleren korpuskularen Volumen der Erythrozyten um 25 % die Messung nicht verfälschen.

Für Hämatokritwerte bis 70 % konnte nachgewiesen werden, daß der Meßwert unabhängig davon ist, ob der Volumenanteil von wenigen großvolumigen oder vielen kleinvolumigen Zellen gebildet wird.

Durch die Wahl dieser Randbedingungen bewirken Osmolaritätsschwankungen im physiologischen Bereich (280 - 300 mosmol) nur eine Schwankung der Plasmaimpedanz von ca. 10 %, was eine Hkt-Wert-Verfälschung um ± 1 % bewirkt.

Es hat sich gezeigt, daß die Bestimmung des Hämatokritwertes innerhalb kürzester Zeit, d.h. innerhalb von ca. einer halben Minute durchgeführt werden kann und daß dieser Meßwert stabil und reproduzierbar ist. Ein Vergleich mit den bisher bekannten Verfahren hat weiterhin gezeigt, daß die erhaltenen Meßwerte sehr genau sind und vor allem nicht mit systematischen Fehlern behaftet sind, da außer dem Auftropfen des Bluttropfens auf die Elektrode und insbesondere deren vollständiger Bedeckung keine weiteren durchzuführenden Manipulationen vorgenommen werden müssen. Die Arbeiten sind sehr gut von einer Hilfskraft durchzuführen. Da sämtliche anderen Meßparameter bereits werksseitig vorgegeben sind, d.h. als eingebaute Meßkonstan-ten (Abstand der Elektroden, spannungskonstanter Oszillator, Festwiderstände, vorgegebene Eichkurve und dgl.), kann eine derartige Vorrichtung selbst beim praktiker eingesetzt werden, und ist somit keineswegs für die Messung im medizinischen Labor vorbehalten.

Selbst wenn versteifte Zellen vorliegen, wird ein exakter Hämatokritwert erhalten, so daß hierdurch keine Störung des Meßablaufs festzustellen ist, was bei den meisten übrigen Verfahren der Fall ist.

Von besonderer Bedeutungist, daß die Sedimentation der zellulären Bestandteile des Blutes keinerlei Auswirkung auf die Hämatokritbestimmung hat und der erhaltene Wert auch nach Minuten immer noch konstant bleibt.

Ausführungsformen der Erfindung sind in der nachfolgenden Beschreibung unter Bezugnahme auf die Zeichnung sowie in den Ausführungsbeispielen beschrieben.

Es zeigen:

Fig. 1 eine schematische Seitenansicht der erfindungsgemäßen Vorrichtung,

Fig. 2 eine schematische Frontansicht der erfindungsgemäßen Vorrichtung,

Fig. 3 eine vergrößerte Teilansicht der Vorrichtung gemäß Fig. 1 und 2,

Fig. 4 eine schematische Schrägansicht der Vorrichtung gemäß Fig. 1 und 2,

Fig. 5 eine Schaltskizze für die erfindungsgemäße Vorrichtung, mit einer Konstantstromquelle.

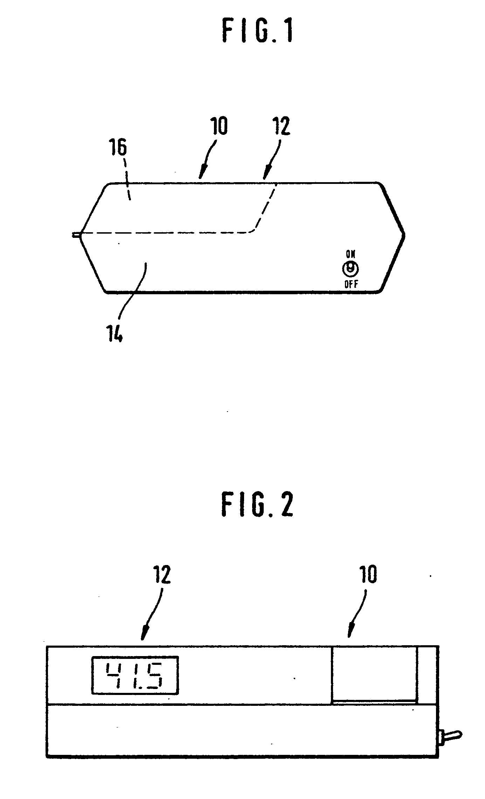

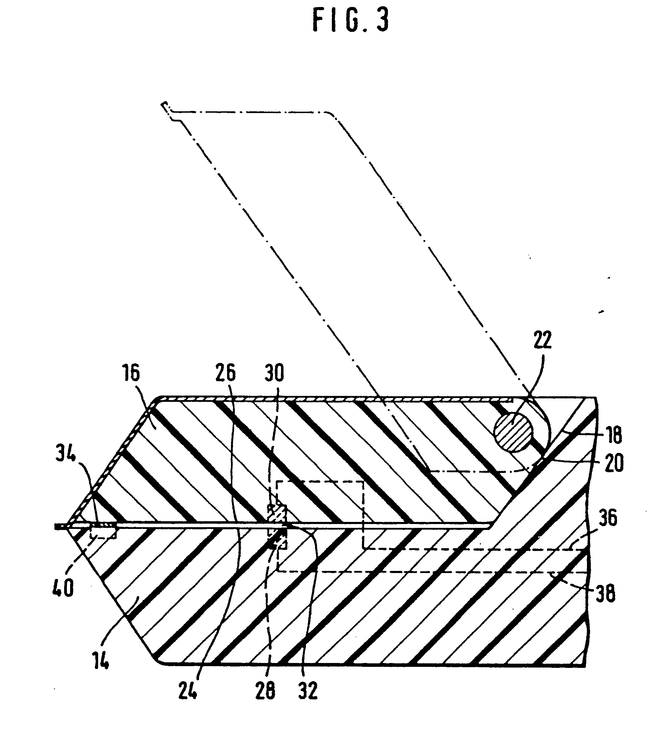

Aus Fig. 1-4 sind die erfindungsgemäße Vorrichtung 10 zur Bestimmung des Hämatokrit und die im selben Gehäuse untergebrachte Registriervorrichtung 12 ersichtlich. Die Vorrichtung 10 besteht im wesentlichen aus einem Unterteil 14 und einem Oberteil 16, das in Form eines Deckels ausgebildet ist und am Unterteil 14 schwenkbeweglich gelagert ist. In der vergrößer-ten Teilansicht von Fig. 3 ist die aufgeklappte Stellung des Oberteils 16 strichpunktiert dargestellt. Die Arretierung des Oberteils selbst erfolgt auf übliche Weise entweder durch Zurückklappen des Deckels oder Oberteils 16, wobei dieser sich an eine Schräge 18 anlegt, die am Unterteil 14 ausgebildet ist, oder durch Festklemmen des Oberteils 16 bei 20 an der Schräge 18. Wie bereits vorstehend festgestellt, ist das Oberteil um die Achse 22 schwenkbar, die das Unterteil 14 und das Oberteil 16 durchsetzt.

Die Oberseite 24 des Unterteils 14 und die Unterseite 26 des Oberteils 16 weisen jeweils eine Elektrode 28 und 30 auf, deren Oberflächen jeweils in der gleichen Ebene wie die Oberseite 24 und Unterseite 26 liegen. Dies hat den Vorteil, daß bei der sehr sorgfältig durchzuführenden Reinigung die gesamte Oberfläche der Ober- und Unterseite vollständig gereinigt werden kann, ohne daß das Meßergebnisse beeinflussende Verschmutzungen zurückbleiben. Andererseits kann jedoch, sofern es zweckmäßig erscheint, die Elektrode 28 und 30 etwas, beispielsweise 1-2 mm vorstehen oder unter die Oberfläche abgesenkt sein.

Die Form und Größe der Elektrodenoberfläche ist nicht kritisch. Zweckmäßigerweise wird man eine runde Elektrodenform wählen, da die Elektrodenoberfläche selbst vollständig von Blut benetzt werden soll, was bei eckigen oder kantigen Formen nicht ohne weiteres möglich ist. Auch die Oberflächengröße der Elektroden 2ß und 30 soll so beschaffen sein, daß das erhaltene Meßsignal sicher und im wesentlichen rauschfrei registriert werden kann, andererseits jedoch nicht zu hohe Blutmengen für die Messung notwendig sind. Üblicherweise liegen die Oberflächengrößen der Elektroden 28 und 30 in einem Bereich von 10-100, insbes. 20-50 mm².

In einer besonders zweckmäßigen Ausführungsform beträgt die Elektrodenoberfläche etwa 30 mm².

Auch das Elektrodenmaterial ist an sich unkritisch. Es ist lediglich zu beachten, daß die Elektroden 28 und 30 gut elektrisch leitend sind und insbesondere keinen Korrosionserscheinungen unterworfen sind oder mit Blut Reaktionen eingehen. Es hat sich herausgestellt, daß die üblicherweise eingesetzten edleren Elektrodenmaterialien, wie Titan, Nickel, Edelstahl, Chrom und die Edelmetalle (Platin, Silber und Gold) selbst, ohne Schwierigkeiten eingesetzt werden können. Aus Kostengründen ist der Einsatz von Edelstahl bevorzugt.

Wie aus der Fig. 3 ersichtlich ist, fluchten die beiden Elektroden 2S und 30 in der Senkrechten, liegen also exakt übereinander, so daß sich im Zwischenraum 32 zwischen den Elektroden 28 und 30 eine zylinderförmig ausgebildete Blutsäule ausbilden kann, an der die elektrische Leitfähigkeit gemessen werden kann. Die Größe des Zwischenraums 32 wird bestimmt durch die Anordnung der Ober- und Unterteile 14 und 16 und insbesondere durch ein Endmaß 34, das als Abstandhalter zwischen der Oberseite 24 und der Unterseite 26 dient. Dabei sind die Oberseite 24 und die Unterseite 26 im geschlossenen Zustand im wesentlichen parallel ausgerichtet, was zu einer ebenfalls parallelen Ausrichtung der Oberflächen der Elektroden 28 und 30 führt.

Es hat sich herausgestellt, daß der Abstand zwischen den Elektroden 28 und 30, d.h. die Höhe des Zwischenraums 32 und damit auch die Dicke des Endmaßes 34 in einem Bereich von etwa 0,5-3, insbesondere 1,4 -2,2 mm liegen kann. In diesem Abstandsbereich wurden mit den vorstehend angegebenen Elektroden-Oberflächenabmessungen eine große Aufspreizung des Meßberei-ches erhalten, wobei Blutmengen von höchstens 500 µl zur Bestimmung des Hämatokrit notwendig sind.

Als Material für die Ober- und Unterseite 24 und 26 werden elektrisch nicht-leitende Materialien, sofern diese mit den Elektroden 28 und 30 in Berührung sind, oder aber übliche metallische Materialien eingesetzt, sofern die Elektroden 28 und 30 von diesen Flächen isoliert sind. Üblicherweise man wird ein thermoplastisches Kunststoffmaterial oder aber die üblichen metallischen Gehäusematerialien für die Vorrichtung 10 einsetzen.

Die Elektroden 28 und 30 sind über Leitungen 36 und 38 mit dem Registriergerät 12 in Verbindung. Dieses Registriergerät tritt in Funktion, wenn vorteilhafterweise ein Endkontakt beim Zuklappen des Oberteils 16 auf das Unterteil 14 geschlossen wird. Vorteilhafterweise ist der Endkontakt 40 mit dem an der Oberseite 16 befindlichen Endmaß 34 in Verbindung zu bringen und läßt sich durch dieses schließen. Über nicht gezeigte Leitungen ist dieser Endkontakt 40 wiederum mit dem Registriergerät 12 in Verbindung.

Den Elektroden 28 und 30 wurde Wechselstrom beispielsweise mit einer effektiven Spannung von 0,1 - 0,7 V insbesondere 0,2 V (bei 0,7 V Gesamtamplitude: 2V) und einer Frequenz von 1,8 - 6, insbesondere 2 - 3 kHz aufgeprägt.

Durch die gewählte Frequenz von 2 - 3 kHz werden polarisationseffekte an den Elektroden unterdrückt. Weiter wird bei dieser Frequenz.der dielektrischen Eigenschaft des plasmas als grobdisperse Lösung großer Dipolmoleküle Lösung getragen. Hierdurch wird der Einfluß der Zellmembranen als Konduktorelemente ausgeschaltet. Andererseits liegt diese Frequenz in einem Bereich in dem die dielektrische Eigenschaft des Plasmas den Meßwert gerade noch beeinflußt. Durch die Wahl des bevorzugten Frequenzbereiches und der bevorzugten Spannung wird erreicht, das sowohl der Impedanzwert des normalen als auch des pathologischen Plasmas, in dem die Eiweißkonzentration unverändert ist, nahezu gleich ist. Ebenfalls wird durch die Wahl der vorstehend erwähnten Frequenzen der Einfluß des kapazitiven Widerstandes der Erythrozytenmembran soweit unterdrückt, daß selbst Schwankungen im mittleren korpuskularen Volumen der Erythrozyten um 25 % die Messung nicht verfälschen.

Bei Hkt-Werten bis 70 % konnte nachgewiesen werden, daß der Meßwert unabhängig davon ist, ob der Volumenanteil von wenigen großvolumigen oder vielen kleinvolumigen Zellen gebildet ist.

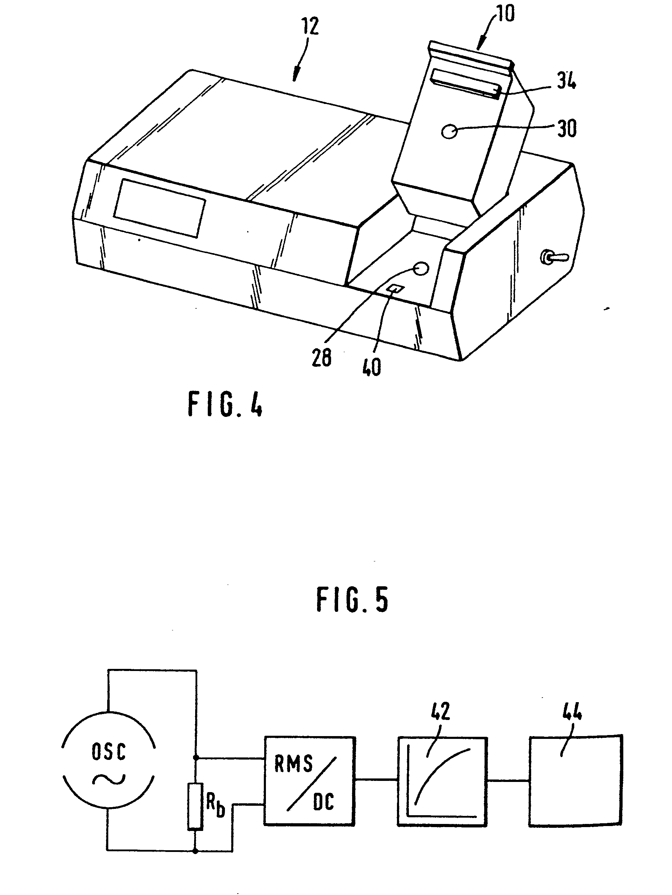

Der vorstehend erwähnte Wechselstrom wird von dem in Fig. 5 gezeigten Oszillator erzeugt. Die Meßspannung selbst wird über der Blutsäule abgegriffen. Nach anschliessender Gleichrichtung des Meßsignals wird dessen Wert mittels einer fest einprogrammierten Eichkurve 42, deren Erstellung nachstehend beschrieben ist, mit den entsprechenden Hämatokritwerten korreliert. Der erhaltene Hämatokritwert wird dann auf einer Anzeige 44, die vorteilhafterweise digital ausgeführt ist, sichtbar auf dem Registriergerät 12 angegeben.

Es muß nicht hervorgehoben werden, daß natürlich die Vorrichtung 10 und das Registriergerät 12 in zwei getrennten Geräten vorliegen können.

Die Messung des Hämatokrits wird folgendermaßen durchgeführt:

Die Meßkammer wird mit etwa 200 µl Blut gefüllt, wobei vor allem auf eine blasenfreie Füllung der Kammer zu achten ist. Dabei kann das Blut einem Patienten an jeder beliebigen, gut zugänglichen Stelle entnommen werden, beispielsweise durch Einstechen in die Fingerbeere und Auftropfen des vorquellenden Blutes auf die untere Elektrode 28. Anschließend klappt man das Oberteil 16 auf das Unterteil 14 und schließt den Endkontakt 40. Nach Einschalten der Stromquelle dauert es etwa 15 Sekunden, bis der angezeigte Spannungsabfall konstant bleibt. Dieser Spannungsabfall wird in erster Linie von der Impedanz, d.h. dem Wechselstromwiderstand, der Erythrozyten bestimmt und ist mit dem Hämatokritwert über eine Eichkurve korreliert. Da die Osmolarität, d.h. die Elektrolytladungseinheiten, eine der im Kreislauf am besten geregelten Größen ist, sind ihre Einflüsse praktisch vernachlässigbar. Erst in zweiter Linie spielen bei der Messung auch der kapazitive Widerstand des Plasmas, der Ohmsche und der kapazitive Widerstand der Erythrozyten eine Rolle. Es hat sich jedoch gezeigt, daß diese Einflüsse als konstant angenommen und daher vernachlässigt werden können.

Der im Registriergerät 12 ankommende Spannungswert ist natürlich abhängig von der Temperatur, der Ionenkonzentration im Suspensionsmedium, dem Elektrodenabstand und bedingt auch dem Füllvolumen der Meßkammer. Wie bereits vorstehend festgestellt, ist der Elektrodenabstand und das Füllvolumen, das sich aus dem Abstand und der Elektrodenoberfläche ergibt, konstant, soferd das Blut die Elektrodenoberfläche vollständig bedeckt. In einer bevorzugten Ausführungsform wird diese vollständige Bedeckung dadurch sichergestellt, daß die Elektroden 30 und 28 an ihrem Außenmaß mit einem Sicherheitsbereich um geben sind, der grundsätzlich mit Blut bedeckt sein muß, wenn die Vorrichtung 10 vom Bedienungspersonal geöffnet wird.

Wie bereits vorstehend, festgestellt, ist die Osmolarität des Plasmas von nachgeordneter Bedeutung für die Bestimmung des Hämatokrits. Sie liegt zwischen Werten von etwa 275 und 300 mosmol/l. Der Meßfehler des Hämatokrit (Hkt) beträgt dann $\Delta$ Hkt - $\pm$ 1 Vol % liegt also bei einem üblichen Hämatokritwert von 40 Vol % in einer Größenordnung von etwa 2,5 %. Aus diesen Gründen kann eine Leermessung an Plasma unterbleiben. Jedoch kann der Leitfähigkeitswert des Plasmas in derselben Kammer bestimmt werden. Gegebenenfülls kann eine Korrektur vorgenommen oder das Gerät selbst kann mit einer Standard NaCl-Lösung geeicht werden.

Die Impedanzmessung zur Hämatokritbestimmung ist natürlich tempraturabhängig, wobei für jede Temperatur eine eigene Eichkurve erstellt werden muß. Insofern ist es vorteilhaft, daß die Vorrichtung 10 für präzisionsmessungen thermostatisiert wird. Diese Thermostatisierung kann auf übliche Weise, beispielsweise durch Beheizung der Vorrichtung 10, erfolgen.

Mißt man bei Zimmertemperatur, so sollte die Impedanz einer gleichtemperierten Referenzlösung bestimmt werden. Der Meß-wert für Blut bei derselben Temperatur muß dann entsprechend korrigiert werden, was in vorteilhafter Weise durch Potentialausgleich mittels eines Potentiometers in der Registriervorrichtung 12 durchgeführt werden kann. Es kann sogar auf eine Temperierung unter diesen Bedingungen verzichtet werden; denn selbst Schwankungen von $\pm$ 3°C bei einer vorgegebenen Zimmertemperatur von etwa 295 K führen nur zu einem Fehler von $\Delta$ Hkt $\pm$ 1 Vol.

## Vergleichsversuch

Erythrozyten wurden mit 5 mmol/l Diamid versteift, wobei mit Hilfe der Zentrifugenmethode ein Hkt-Wert von 0,35 und 0,51 eingestellt wurde. Anschließend wurde der Hämatokritwert nach der erfindungsgemäßen Impedanzmethode und mit Hilfe der Radiohämatokritmethode, also mit markierter $^{14}$C-Saccharose bestimmt. Die hierbei ermittelten Werte ergaben eine 1:1 Korrelation zwischen den letztgenannten beiden Meßmethoden, wobei ein Wert von jeweils etwa 32,4 und 46,0 gefunden wurde. Gegenüber diesen Werten lag der mit der Zentrifugationsmethode bestimmte Wert um 6-7 % (Hkt=0,35) und um 10-11 % (Hkt=0,51) höher.

Insgesamt gesehen ist mit der erfindungsgemäßen Vorrichtung eine Hämatokritmessung im Bereich von 0-80 Vol % möglich. Durch Vollautomatisierung ist eine einfache Bedienung möglich. Die Gesamtmeßzeit liegt unter 30 Sekunden. Außerdem ist die Patientenbelastung gering, da nur etwa 70 - 200 µl Blut benötigt werden.

## Patentansprüche:

1. Vorrichtung zur Bestimmung des Hämatokritwertes zwischen zwei Elektroden (28,30), deren Meßflächen parallel zur Horizontalebene angeordnet sind und mit denen die Leitfähigkeit von Blut bestimmbar ist, mit einem Unterteil (14) und einem Oberteil (16), die auseinanderbewegbar sind und eine horizontale Kammer bilden, dadurch gekennzeichnet, daß wenigstens eine Elektrode (30) in der Ebene der Unterseite (26) des Oberteils (16) und eine Elektrode (28) in der Ebene der Oberseite (24) des Unterteils (14) angeordnet sind, und daß die Elektroden (28,30) in der Senkrechten fluchten.

2. Vorrichtung nach Anspruch 1, dadurch gekennzeichnet, daß das Unterteil (14) und das Oberteil (16) um eine Achse (22) drehbar gelagert sind.

3. Vorrichtung nach Anspruch 1, dadurch gekennzeichnet, daß die Elektroden (28,30) eine im Wesentlichen runde Form und eine Fläche von etwa 10 - 100 mm² aufweisen.

4. Vorrichtung nach Anspruch 3, dadurch gekennzeichnet, daß die Fläche 20 - 50 mm² beträgt.

5. Vorrichtung nach Anspruch 1, dadurch gekennzeichnet, daß die Elektroden (28, 30) aus Edelstahl, Titan oder einem anderen edlen Elektrodenmaterial bestehen.

6. Vorrichtung nach Anspruch 1, dadurch gekennzeichnet, daß die Elektroden (28, 30) aus einem unedlen Material, das mit einer Beschichtung aus den Materialen des Anspruches 5 versehen ist, besteht.

7. Vorrinctung nach Anspruch 1, dadurch gekennzeichnet, daß der Abstand den Elektroden (28, 30) etwa 0,5 - 3 mm beträgt.

8. Vorrichtung nach Anspruch 7, dadurch gekennzeichnet, daß der Abstand etwa 1,4 - 2,2 mm beträgt.

9. Vorrichtung nach Anspruch 1, gekennzeichnet durch einen Endkontakt (40), mit die Vorrichtung (10) in den Meßzustand versetzbar ist.

10. Vorrichtung nach Anspruch 9, dadurch gekennzeichnet, daß ein Endmaß (34) vorgesehen ist, das als Abstandshalter zwischen dem Oberteil (16) und dem Unterteil (14) und als Kontakt des Endkontakts (40) dient.

11. Vorrichtung nach Anspruch 1, dadurch gekennzeichnet, daß sie thermostatisierbar ist.

12. Vorrichtung nach Anspruch 1, dadurch gekennzeichnet, daß an die Elektroden (28,30) im Betriebszustand mittels eines Oszillators eine Wechselspannung anlegbar ist, wobei ein Vorwiderstand in Reihe schaltbar und/oder ein Wechselstrom von 0,1 - 0,7 V aufprägbar ist.

13. Vorrichtung nach Anspruch 12, dadurch gekennzeichnet, daß eine Frequenz von 1,8 - 6 kHz aufprägbar ist.

**Claims:**

1. Apparatus for the determination of the haematocrit value between two electrodes (28, 30) whose measuring surfaces are arranged parallel to the horizontal plane and with which the conductivity of blood can be determined, having a lower part (14) and an upper part (16), which can be moved apart and which form a horizontal chamber, characterised in that at least one electrode (30) is located in the plane of the underside (26) of the upper part (16) and one electrode (28) is located in the plane of the upper side (24) of the lower part (14), and in that the electrodes (28, 30) are aligned vertically.

2. Apparatus according to Claim 1, characterised in that the lower part (14) and the upper part (16) are mounted to be rotatable about an axis (22).

3. Apparatus according to Claim 1, characterised in that the electodes (28, 30) have an essentially circular shape and an area of about 10-100 mm²

4. Apparatus according to Claim 3, characterised in that the area is 20-50 mm².

5. Apparatus according to Claim 1, characterised in that the electrodes (28, 30) are composed of special steel, titanium or another inert electrode material.

6. Apparatus according to Claim 1, characterised in that the electrodes (28, 30) are composed of a non-inert material which is provided with a coating of the materials of Claim 5.

7. Apparatus according to Claim 1, characterised in that the distance between the electrodes (28, 30) is about 0.5-3 mm.

8. Apparatus according to Claim 7, characterised in that the distance is about 1.4-2.2 mm.

9. Apparatus according to Claim 1, characterised by a terminal contact (40) with which the apparatus (10) can be put in the state for measurement.

10. Apparatus according to Claim 9, characterised in that a gauge block (34) is provided, which acts as a spacer between the upper part (16) and the lower part (14) and as a contact of the terminal contact (40).

11. Apparatus according to Claim 1, characterised in that it can be thermostated.

12. Apparatus according to Claim 1, characterised in that an alternating voltage can be applied by means of an oscillator to the electrodes (28, 30) in the operating state, it being possible for a drop resistance to be connected in series and/or an alternating current of 0.1-0.7 V to be impressed.

13. Apparatus according to Claim 12, characterised in that a frequency of 1.8-6 kHz can be impressed.

**Revendications**

1. Appareil pour déterminer l'hématocrite entre deux électrodes (28, 30) dont les surfaces de mesure sont disposées parallélément à un plan horizontal et au moyen desquelles on peut déterminer la conductivité du sang, l'appareil comprenant une partie inférieure (14) et une partie supérieure (16) qui peuvent être écartées l'une de l'autre et forment ensemble une chambre horizontale, caractérisé en ce qu'au moins une électrode (30) est disposée dans le plan du dessous (26) de la partie supérieure (16) et au moins une électrode (28) est disposée dans le plan du dessus (24) de la partie inférieure (14), et en ce que les électrodes (28, 30) sont mutuellement alignées verticalement.

2. Appareil selon la revendication 1, caractérisé en ce que la partie inférieure (14) et la partie supérieure (16) sont montées de façon à pouvoir être tournées l'une par rapport à l'autre autour d'un axe (22).

3. Appareil selon la revendication 1, caractérisé en ce que les électrodes (28, 30) ont une forme essentiellement ronde et possèdent une aire de surface d'environ 10-100 mm$^2$.

4. Appareil selon la revendication 3, caractérisé en ce que l'aire de surface est de 20 - 50 mm$^2$

5. Appareil selon la revendication 1, caractérisé en ce que les électrodes (28, 30) sont en acier fin, titane ou un autre matériau noble pour électrodes.

6. Appareil selon la revendication 1, caractérisé en ce que les électrodes (28, 30) sont réalisées en un matériau commun portant un revêtement d'un des matériaux de la revendication 5.

7. Appareil selon la revendication 1, caractérisé en ce que la distance entre les électrodes (28, 30) est d'environ 0,5 - 3 mm.

8. Appareil selon la revendication 7, caractérisé en ce que la distance entre les électrodes est d'environ 1,4 - 2,2 mm.

9. Appareil selon la revendication 1, caractérisé en ce cu'il comprend un interrupteur de fin de course (40) au moyen du quel l'appareil (10) peut être amené à l'état de mesure.

10. Appareil selon la revendication 9, caractérisé en ce qu'il comprend une butée (34) qui sert d'entretoise entre la partie supérieure (16) et la partie inférieure (14) et de contact ou d'élément de commande de l'interrupteur de fin de course (40).

11. Appareil selon la revendication 1, caractérisé en ce que sa température est réglable automatiquement.

12. Appareil selon la revendication 1, caractérisé en ce que les électrodes (28, 30) reçoivent, pendant le fonctionnement, une tension alternative fournie par un oscillateur, avec montage en série d'une résistance et/ou avec application d'une tension alternative de 0,1 - 0,7 V.

13. Appareil selon la revendication 12, caractérisé par l'application d'une tension alternative d'une fréquence de 1,8-6 kHz.

# FIG.1

# FIG.2

# FIG.3

FIG. 4

FIG. 5